# EUROPEAN PATENT APPLICATION

(11) **EP 4 722 713 A1**
(43) Date of publication of application: **08.04.2026**
(21) Application number: 24811133.8
(22) Date of filing: 22.05.2024
(51) Int. Cl.: G01N 30/88, G01N 30/86, G01N 33/72

(54) **METHOD OF ANALYZING HEMOGLOBIN AND SYSTEM FOR ANALYZING HEMOGLOBIN**

(30) Priority: 24.05.2023 JP 2023085130
(71) Applicant: TOSOH CORPORATION, Shunan-shi, Yamaguchi 746-8501 (JP)
(72) Inventor: MANITA, Daisuke, Ayase-shi, Kanagawa 252-1123 (JP); OGINO, Shinji, Shunan-shi, Yamaguchi 746-8501 (JP)
(74) Representative: Novagraaf International SA
(86) International application number: PCT/JP2024/018778
(87) International publication number: WO 2024/242127

(57) **Abstract**

The present invention provides a method for analyzing hemoglobin in a blood-derived sample, the method including: (1) a step for identifying a first peak with regard to HbAlc and a second peak with regard to HbA0; (2) a step for discerning whether there is furthermore a third peak present between the first peak and the second peak. The following steps are also implemented if it is discerned that the third peak is present and no other peak besides the third peak is present: (3) a step for calculating the slope of a straight line formed between a first valley present between the first peak and the third peak, and a second valley present between the second peak and the third peak; and (4) a step for estimating the presence of abnormal hemoglobin in the sample from a comparison of the slope and a predetermined reference threshold value range. Also provided is a system for implementing the method.

## Description

### FIELD

The present invention relates to a method for analyzing hemoglobin and to a system for analyzing hemoglobin. More specifically, the invention relates to a method and system for measuring stable HbAlc in a blood sample, which allow estimation of an abnormal hemoglobin-containing specimen by cation-exchange liquid chromatography, and accurate reporting of the measurement results.

### BACKGROUND

Hemoglobin A1c (HbA1c, SA1c, or stable HbA1c) is a biomarker that nonenzymatically binds with glucose in the blood (blood glucose) or its metabolites, and it is widely used for diabetes diagnosis and treatment monitoring.

Measurement of HbA1c is widely carried out based on the principle of cation-exchange liquid chromatography (CEX-HPLC). Methods of measuring HbAlc by CEX-HPLC have the advantage of allowing detection of hemoglobin fractions caused by mutations in the hemoglobin gene, collectively known as abnormal hemoglobin, but also have a disadvantage in that the presence of the latter can potentially interfere with precision of the results for HbA1c.

Hb-Riccarton is a type of abnormal hemoglobin that is widely found mainly in Europe. NPL 1 reports that the results of HbAlc measurement by CEX-HPLC may be underevaluated when measuring Hb-Riccarton-containing specimens.

Moreover, as demonstrated in PTL 1, when a blood sample containing hemoglobin E (HbE), a different type of abnormal hemoglobin, is measured by cation-exchange chromatography, a peak attributable to HbE appears between stable HbAlc and HbA0.

Since the peaks due to Hb-Riccarton also appear at the same location as HbE, as indicated in NPL 1, it has been difficult to discriminate HbE and Hb-Riccarton by measurement of HbA1c using cation-exchange chromatography. Considering the relative prevalence of HbE and Hb-Riccarton, HbE cases are overwhelmingly more common, making it highly probable that Hb-Riccarton is being reported as HbE.

### [CITATION LIST]

### [PATENT LITERATURE]

[PTL 1] Japanese Unexamined Patent Publication No. 2012-215470

### [NON PATENT LITERATURE]

[NPL 1] Brennan, Stephen O., et al. Hb Riccarton [α51 (CE9) Gly→ Ser]: a variant arising from a novel mutation in the α1 gene. Hemoglobin 29.1 (2005): 61-64.

### SUMMARY

### [TECHNICAL PROBLEM]

In specimens where a peak is observed between the HbA1c peak and the HbA0 peak and a different abnormal hemoglobin from HbE (such as Hb-Riccarton) is present, the measurement results for HbA1c may not be accurate. It is an object of the invention to develop a method and system that allow estimation of the presence of abnormal hemoglobin even when measuring specimens as described above by cation-exchange liquid chromatography.

### [SOLUTION TO PROBLEM]

As a result of much research on this problem, the present inventors have completed this invention upon finding that it is possible to estimate the presence of abnormal hemoglobin from a chromatogram of a sample containing multiple hemoglobins including HbA1c and HbA0. Specifically, the invention has the following aspects.

[1] A method for analyzing hemoglobin from a blood-derived sample, the method comprising:
   (1) a step of identifying a first peak for HbAlc and a second peak for HbA0 from a chromatogram representing elution of a plurality of hemoglobin fractions including at least HbA1c and HbA0, obtained by supplying a blood-derived sample to cation-exchange liquid chromatography;
   (2) a step of discriminating whether or not an additional third peak is present between the first peak and the second peak, the following steps being further carried out when it has been judged that a third peak is present and no peaks other than the third peak are present;
   (3) a step of identifying a first trough between the first peak and the third peak, and a second trough between the second peak and the third peak, and calculating the slope of a straight line formed between the first trough and the second trough; and
   (4) a step of estimating the presence of abnormal hemoglobin in the sample based on comparison between the slope and a predetermined reference threshold range.
[2] The method according to [1] above, wherein in step (3), the slope is calculated from the local minimum of the first trough and the local minimum of the second trough.
[3] The method according to [1] or [2] above, wherein in step (4), it is estimated that abnormal hemoglobin different from hemoglobin E is present in the sample when the slope has deviated from the predetermined reference threshold range.
[4] The method according to [3] above, wherein, when it has been estimated that abnormal hemoglobin different from hemoglobin E is present in the sample, one or more steps are carried out from among the following steps:
   (a) the response given is that the measurement results for HbAlc for the sample cannot be reported;
   (b) the response given is the measurement results for HbAlc for the sample, with an associated identifier indicating the presence of the abnormal hemoglobin different from hemoglobin E; and
   (c) the area of the third peak is included in the area of the first peak to calculate HbA1c, and the response given is the measurement results.
[5] The method according to [4] above, wherein the abnormal hemoglobin different from hemoglobin E is Hb-Riccarton.
[6] The method according to [1] or [2] above, wherein in step (4), it is estimated that hemoglobin E is present in the sample when the slope is within the predetermined reference threshold range.
[7] The method according to [6] above,
   wherein, when it is estimated that hemoglobin E is present in the sample, HbAlc is calculated by correcting hemoglobin E using the area of the third peak, and the response given is the measurement results.
[8] The method according to any one of [1] to [7] above, wherein the chromatogram is a chromatogram showing time on the abscissa and detector output on the ordinate, and the predetermined reference threshold range is -30 to 30, where the minimum detector output during measurement is normalized to 0 and the maximum value is normalized to 1000 in the chromatogram.
[9] The method according to any one of [1] to [8] above, wherein the predetermined reference threshold range is calculated by steps including the following steps:
   (i) a step of identifying a first peak for HbA1c, a second peak for HbA0 and a third peak for hemoglobin E, from a chromatogram representing elution of a plurality of hemoglobin fractions including at least HbA1c, HbA0 and hemoglobin E, obtained by supplying a blood-derived sample of a hemoglobin E-containing reference specimen to cation-exchange liquid chromatography,
   (ii) a step of identifying a first trough between the first peak and the third peak and a second trough between the second peak and the third peak, and calculating a reference slope from the local minimum of the first trough and the local minimum of the second trough; and
   (iii) a step of carrying out (i) to (ii) for multiple hemoglobin E-containing reference specimens, and calculating the predetermined reference threshold range by statistical processing from the obtained values for the multiple reference slopes.
[10] A hemoglobin analysis system in which the method according to any one of [1] to [9] above is carried out.
[11] The hemoglobin analysis system according to [10] above, which comprises:
   a cation-exchange liquid chromatography apparatus, and
   analyzing means for analyzing chromatogram data obtained by the cation-exchange liquid chromatography apparatus.

### [ADVANTAGEOUS EFFECTS OF INVENTION]

With the HbAlc measuring apparatus of the invention having the construction described above, it is possible to determine that measurement results cannot be properly reported, or to report accurate measurement results, even when measuring a specimen wherein a peak is observed between the HbAlc peak and the HbA0 peak and abnormal Hb different from HbE (such as Hb-Riccarton) is present.

### BRIEF DESCRIPTION OF DRAWINGS

Fig. 1 is a block diagram of a measuring apparatus according to an embodiment of the disclosure.
Fig. 2 is a flow chart showing an example of analysis processing in a measuring apparatus.
Fig. 3 is an example of a chromatogram for a common specimen measured with the measuring apparatus of Fig. 1.
Fig. 4 is an example of a chromatogram for an HbE specimen measured with the measuring apparatus of Fig. 1.
Fig. 5 is an example of a chromatogram for an Hb-Riccarton specimen measured with the measuring apparatus of Fig. 1.
Fig. 6 shows a magnified view of the chromatogram of the HbE specimen of Fig. 4, with an auxiliary line indicating the slope.
Fig. 7 shows a magnified view of the chromatogram of the Hb-Riccarton specimen of Fig. 5, with an auxiliary line indicating the slope.
Fig. 8 is a histogram showing distribution of the slopes of troughs (82 measurements) forming an HbE specimen peak.

### DESCRIPTION OF EMBODIMENTS

The present invention will now be described in greater detail by Examples, with the understanding that the invention is not limited to the Examples. The entirety of the documents cited throughout the present specification may be incorporated herein by reference.

According to one aspect, the invention provides a method for analyzing hemoglobin from a blood-derived sample, the method comprising:
(1) a step of identifying a first peak for HbAlc and a second peak for HbA0 from a chromatogram representing elution of a plurality of hemoglobin fractions including at least HbAlc and HbA0, obtained by supplying a blood-derived sample to cation-exchange liquid chromatography;
(2) a step of discriminating whether or not an additional third peak is present between the first peak and the second peak, the following steps being further carried out when it has been judged that a third peak is present and no peaks other than the third peak are present;
(3) a step of identifying a first trough between the first peak and the third peak, and a second trough between the second peak and the third peak, and calculating the slope of a straight line formed between the first trough and the second trough; and
(4) a step of estimating the presence of abnormal hemoglobin in the sample based on comparison between the slope and a predetermined reference threshold range.

According to another embodiment, the invention provides a hemoglobin analysis system in which the method described above is carried out. The hemoglobin analysis system provided by the invention may comprise analyzing means for analyzing data from a chromatogram acquired by a cation-exchange liquid chromatography apparatus, and for example, it may be a computer in which a control program for carrying out the method is installed, where the computer comprises a processor (such as a CPU or microcomputer). According to another aspect, the invention may be a computer program that causes the analyzing means to carry out the method described above.

The data for the chromatogram acquired from the cation-exchange liquid chromatography apparatus may be introduced into the analyzing means through a network (wired network or wireless network) or a storage medium (for example, a memory device such as a RAM, ROM or flash memory, a fixed disk device such as a hard disk drive, or a portable storage device such as a flexible disk or optical disk).

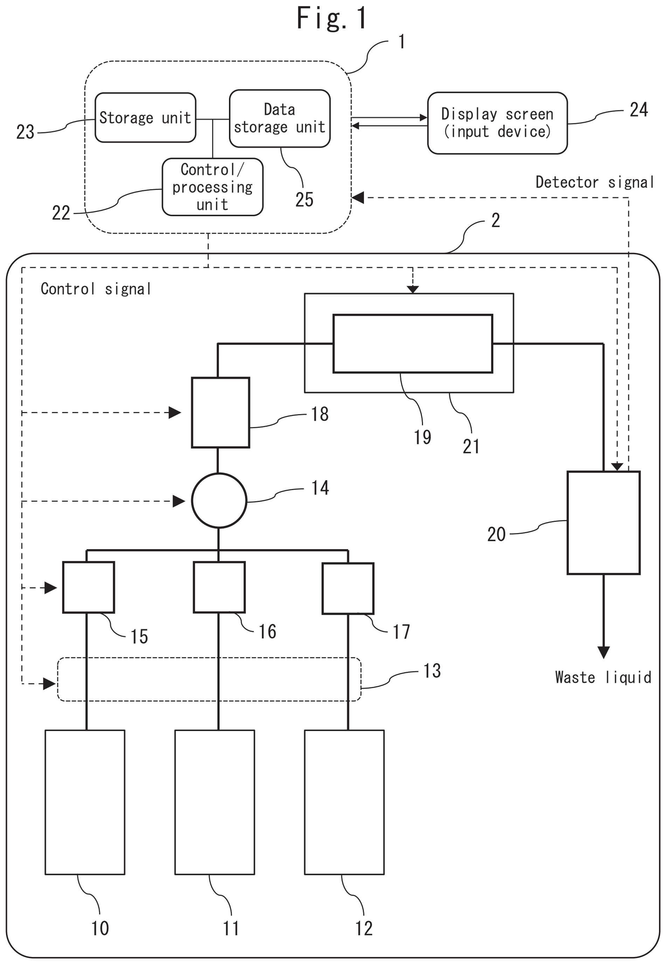

According to another aspect, the invention may comprise a cation-exchange liquid chromatography apparatus and analyzing means for analyzing chromatogram data obtained by the cation-exchange liquid chromatography apparatus (see Fig. 1, for example). In this case, the computer used as the analyzing means may be in a form incorporated into the cation-exchange liquid chromatography apparatus in a non-separable manner, or it may be provided in a so-called "external" form.

The cation-exchange liquid chromatography to be applied for the invention will now be described. A cation-exchange column is generally prepared from a filler having a cation-exchange group introduced into non-porous crosslinked polymer particles, but the method of synthesizing the non-porous crosslinked polymer particles is not restricted and may be a known suspension polymerization method, emulsion polymerization method, seed polymerization method or precipitation polymerization method. According to the invention, there is no limitation to non-porous crosslinked polymer particles, and a porous crosslinked polymer may be used. There are no particular restrictions on the monomers and crosslinking agents used for synthesis, but hydrophilic methacrylic acid and acrylic esters are preferred. Monomers include hydroxyethyl methacrylate, hydroxyethyl acrylate, hydroxypropyl acrylate, glycerol methacrylate, polyethylene glycol methacrylate and polyethylene glycol acrylate. Crosslinking agents include ethylene glycol dimethacrylate, diethylene glycol dimethacrylate, triethylene glycol dimethacrylate and glycerol dimethacrylate.

When non-porous crosslinked polymer particles are used, there are no particular restrictions on the method of introducing the cation-exchange group on the surface, and any publicly known introduction method may be used. Cation-exchange groups that may be introduced include sulfopropyl, sulfoethyl and carboxylmethyl groups. The same examples may be mentioned as for use of a porous crosslinked polymer.

There are no particular restrictions on the eluent used for cation-exchange liquid chromatography according to the invention, and it may be a buffering solution comprising an organic acid such as succinic acid or citric acid and its salt, a buffering solution comprising an inorganic acid such as phosphoric acid and its salt, or a mixture of both an organic acid and an inorganic acid. If necessary, other salts such as sodium chloride, sodium nitrate or sodium sulfate may be added to the buffering solution.

In the Examples, the cation-exchange liquid chromatography apparatus used to obtain the chromatogram representing elution of the hemoglobin fraction was an HLC-723GR01 Automatic Glycohemoglobin Analyzer (standard short mode) (Tosoh Corp.), but there is no limitation to this device. This apparatus is for liquid chromatography based on the principle of cation-exchange chromatography. The main system configuration of the apparatus will now be explained with reference to Fig. 1.

The analysis column (19) used was TSKgel GR01 (standard short mode) (Tosoh Corp.), and the eluents used were a GR01 eluent first solution (10), a GR01 eluent second solution (11) and a GR01 eluent third solution (12). These measuring conditions allow measurement with a measuring time of 30 seconds per specimen.

Each eluent (10, 11, 12) is conveyed by a liquid feed pump 14 and delivered to an analysis column 19 together with the measuring sample injected by the autosampler 18. Each eluent (10, 11, 12) is passed through a degasser 13 as necessary, creating a different eluent mixing ratio by switching of solenoid valves (15, 16, 17), the components being separated with an analysis column and the fractions being successively eluted, depending on the difference in salt concentration created by the mixing ratio. The successively eluted fractions are guided to a detector 20 and the output value is continuously determined.

The degasser 13, solenoid valves (15, 16, 17), liquid feed pump 14, autosampler 18, column oven 21 and detector 20 are controlled by a control/processing unit 22 which is a microcomputer or CPU, based on the control program and measuring conditions prestored in a storage unit 23. The detector signal obtained by the detector 20 is converted by the control/processing unit 22 into plot data comprising time and a detector signal, for example. This plot data is then used to draw a chromatogram. The chromatogram is further processed by waveform processing and the HbA1c is quantitatively calculated based on previously stored calibration data. The obtained quantitative calculation results and chromatogram may be displayed on a display screen 24, allowing the data to be stored in a data storage unit 25.

According to one aspect of the invention, a chromatogram is acquired based on plot data with time (retention time) [t] on the abscissa and detector output (signal output) [A] such as absorbance on the ordinate, but elution volume may also be plotted on the abscissa. The ordinate may also represent the value calculated from the detector signal. Correction such as noise processing, smoothing, drift processing or baseline compensation, for example, may also be carried out. The method of displaying the chromatogram may be normalization, to allow comparison while excluding the effects of the measuring conditions and environmental conditions.

According to one aspect of the invention, normalization may be carried out, whereby the minimum value for the detector output in plot data for one measurement is defined as 0, the maximum value for the detector output is defined as 1000, and the other points are multiplied by a constant so that their relative proportions remain the same. The normalization conditions are for relative evaluation of specific peak heights across different measurements or specimens, and they may be based on any arbitrary time point or arbitrary peak top, or alternatively, the normalization may be carried out with respect to the abscissa.

Calculation of HbAlc is generally based on the ratio of the area of the HbA1c peak with respect to the total hemoglobin area (total area), in a chromatogram obtained by fractionation of a hemoglobin-containing sample. Calibration is usually carried out based on measurement results for known samples, in order to correct for deviations from true or reference values due to the measuring method or measuring conditions. Means exist for removing the peak areas of peaks that affect the total area, or correcting the results before and after calibration, in order to eliminate abnormal hemoglobin or modified hemoglobin that can affect HbAlc measurement results. For example, when hemoglobin E is estimated to be present in a sample according to the invention, for measurement of HbA1c which includes abnormal hemoglobin E, the HbAlc measurement results can be obtained by identifying peaks for abnormal hemoglobin E as peaks appearing between the peak for non-glycated hemoglobin A and the peak for sA1c, calculating the area of the peaks for abnormal hemoglobin E, and using the calculation results to correct the peak area for HbA1c or the peak area for total hemoglobin, as described in PTL 1 (Japanese Unexamined Patent Publication No. 2012-215470).

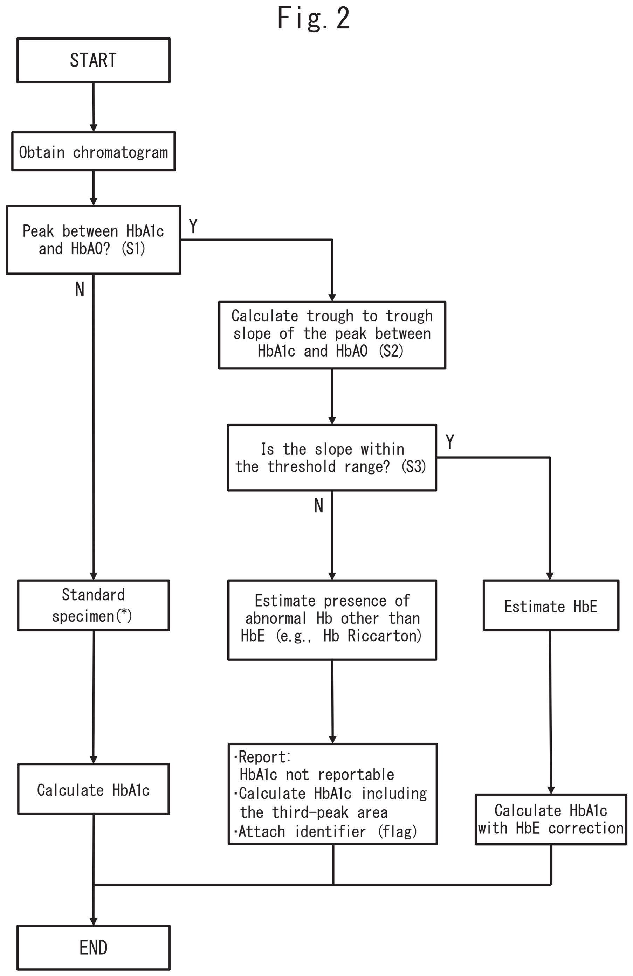

Fig. 2 is an example of a flow chart illustrating the method of the invention according to one aspect. An example of abnormal hemoglobin that can be estimated in this flow chart is Hb-Riccarton, a type of abnormal Hb different from HbE, which has a peak (corresponding to the third peak) between the HbAlc peak (corresponding to the first peak) and the HbA0 peak (corresponding to the second peak).

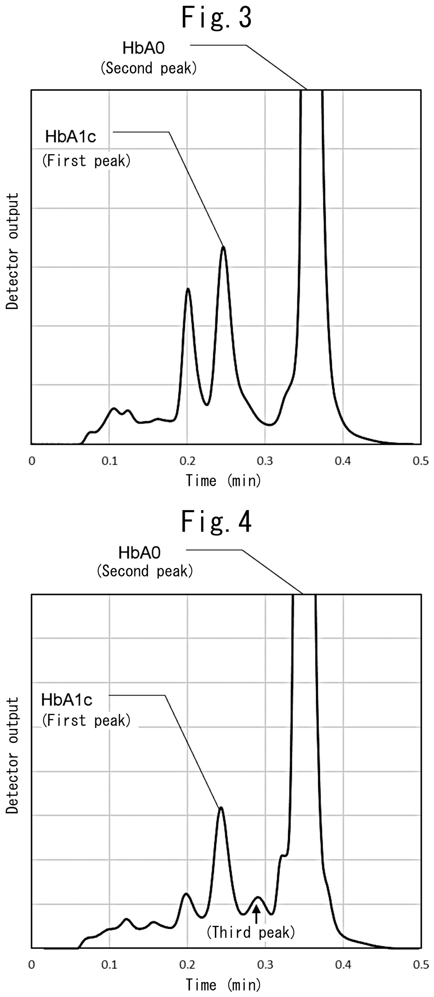

Fig. 3 shows an example of a chromatogram obtained by HbA1c measurement of a common specimen. An HbA1c peak (corresponding to the first peak) and an HbA0 peak (corresponding to the second peak) were observed, while no peak was observed between the HbAlc and HbA0 peaks.

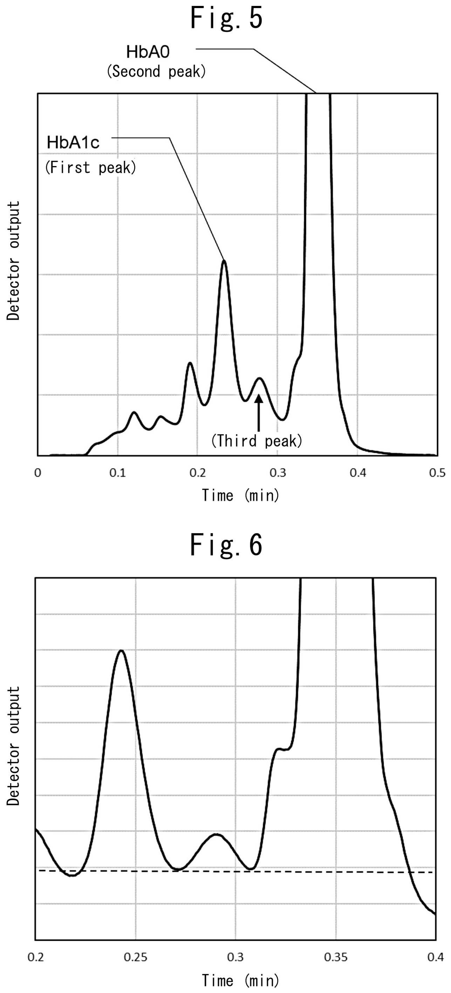

Fig. 4 shows an example of a chromatogram obtained by HbAlc measurement of a specimen containing hemoglobin E, and Fig. 5 shows an example of a chromatogram obtained by HbA1c measurement of a specimen containing Hb-Riccarton. It is seen that hemoglobin E and Hb-Riccarton both exhibit a peak (corresponding to the third peak) between HbAlc and HbA0, with no other abnormal peaks. While the details regarding hemoglobin E and Hb-Riccarton remain unknown, various studies have demonstrated that they have similar elution positions regardless of cation-exchange groups and eluent conditions, and it has been found that separating them involves extending the measuring time or impairing the elution conditions for the other peaks. When they are compared based only on the difference in elution time, misidentification tends to occur due to differences between apparatuses, differences between measuring days and differences between measuring conditions.

A flow chart illustrating one aspect of the invention will now be explained. In a chromatogram obtained by analyzing a measuring sample, it is first judged whether an additional separate peak (third peak) is present between the HbAlc peak (first peak) and the HbA0 peak (second peak), and whether no other abnormal peak is present (S1). When a peak cannot be obtained between the peaks for HbAlc and HbA0, it is judged that there exists no hemoglobin E or abnormal Hb (such as Hb-Riccarton) with a peak between the HbA1c peak and the HbA0 peak. Such specimens can be specimens other than standard specimens, with abnormal Hb exhibiting a peak other than between the HbAlc and HbA0 peaks, but for this embodiment, any abnormal Hb specimen exhibiting a peak other than between the HbAlc and HbA0 peaks will be ignored and listed as a standard specimen(*) (Fig. 2). When a peak (third peak) is obtained between the peaks for HbAlc and HbA0, the slopes of the troughs for that peak are calculated (S2). The method of determining the slope may be calculation of the ratio between changes of two different points between the HbA1c peak (first peak) and the HbA0 peak (second peak), or the chromatogram peaks may be applied to a continuous function to determine the slope of the tangent line at any arbitrary point. From the viewpoint of reducing calculation processing, it is preferred to calculate the slope of each trough (local minimum) formed sandwiching the top of the peak. For example, if the trough between the first peak and the third peak is identified as the "first trough" and the trough between the second peak and the third peak is identified as the "second trough", with the plot data for the earlier eluting local minimum (i.e. the "local minimum of the first trough") as (t1, A1) and the plot data for the later eluting local minimum (i.e. the "local minimum of the second trough") as (t2, A2), the slope which is the ratio of their change may be represented as (A2 - A1)/(t2 - t1).

The predetermined reference threshold range that has been preset and stored in the HbA1c measuring apparatus is compared with the value for this slope (S3). When the comparison shows that the slope deviates from the established reference threshold value range, it may be estimated that abnormal Hb different from HbE (such as Hb-Riccarton) is present. When the slope is within the predetermined reference threshold range, on the other hand, it may be estimated that HbE is present. The predetermined reference threshold range may be set during production and sale of the system of the invention, or a user may newly set and modify it. During measurement of a known HbE specimen, numerical values may be stored and the value set and updated from the average value, center value, median value, standard deviation or dispersion, or from statistical processing based on the same. For example, according to one embodiment the predetermined reference threshold range may be calculated by steps including the following steps:
(i) a step of identifying a first peak for HbA1c, a second peak for HbA0 and a third peak for hemoglobin E, from a chromatogram representing elution of a plurality of hemoglobin fractions including at least HbA1c, HbA0 and hemoglobin E, obtained by supplying a blood-derived sample of a hemoglobin E-containing reference specimen to cation-exchange liquid chromatography,
(ii) a step of identifying a first trough between the first peak and the third peak and a second trough between the second peak and the third peak, and calculating a reference slope from the local minimum of the first trough and the local minimum of the second trough; and
(iii) a step of carrying out (i) to (ii) for multiple hemoglobin E-containing reference specimens, and calculating the predetermined reference threshold range by statistical processing from the obtained values for the multiple reference slopes.

When it has been estimated according to the invention that abnormal Hb different from HbE (such as Hb-Riccarton) is present, a response may be given that calculation of HbA1c in the presence of HbE (for example, the method described in PTL 1) is not carried out, or that the measurement results for HbAlc cannot be reported.

An identifier such as a message or flag may also be attached to the measurement results, suggesting the presence of abnormal hemoglobin different from HbE (such as Hb-Riccarton).

According to another aspect, when it has been estimated that abnormal Hb different from HbE (such as Hb-Riccarton) is present, the same HbAlc calculation is not carried out as when HbE is present, but HbAlc may be calculated in a manner corresponding to the presence of abnormal Hb different from HbE (such as Hb-Riccarton), allowing HbAlc to be reported. As an example of such a method, the area of the third peak may be included in the area of the first peak to calculate HbA1c, and the measurement results reported.

Abnormal hemoglobin Hb-Riccarton is hemoglobin having an α-globin chain mutation in which glycine (Gly) as the amino acid at position 51 of the α-globin chain, one of the subunits composing hemoglobin, is mutated to serine (Ser) (see NPL 1, for example).

### [Examples]

The present invention will now be described in greater detail by Examples and Comparative Examples, with the understanding that the invention is not limited to the Examples.

### (Example 1)

HbE specimens and Hb-Riccarton were obtained from Analytical Control Systems, The European Reference Laboratory for Glycohemoglobin, Eurofins. Each specimen was used for gene analysis at the Fukuyama Rinsho Examination Center.

Fig. 6 is a magnified view of the peak between the peak for HbAlc and the peak for HbA0 in an HbE specimen, where the dotted line is an auxiliary line representing the slope at chromatogram coordinate points at the two trough points forming the peak.

During this time, the deviation Δt = 0.0354 during the time between the two points (abscissa), and the deviation ΔA = 0.03 for the detector output (ordinate), for a slope ΔA/Δt = 0.85.

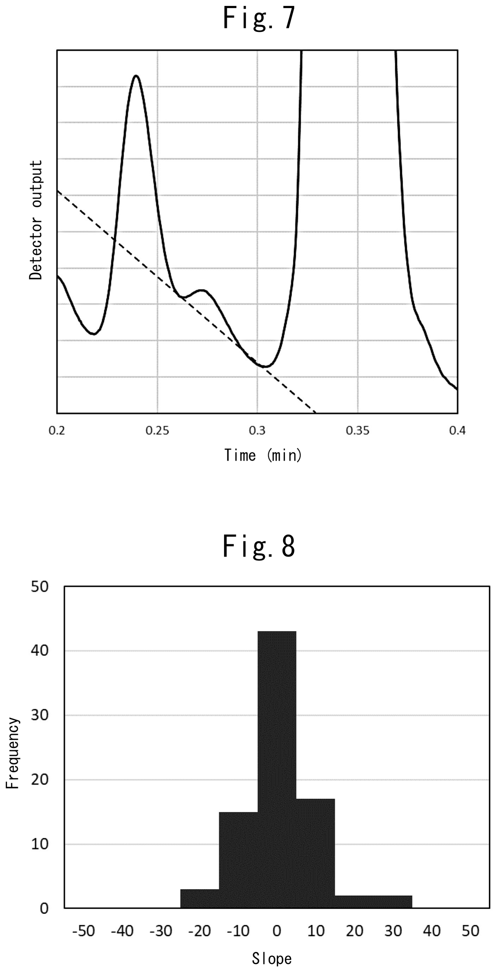

Fig. 7 is a magnified view of the peak between the peak for HbAlc and the peak for HbA0 in an HbE specimen, where the dotted line is an auxiliary line representing the slope at chromatogram coordinate points at the two trough points forming the peak.

During this time, the deviation Δt = 0.0417 during the time between the two points (abscissa), and the deviation ΔA = -3.816 for the detector output (ordinate), for a slope ΔA/Δt = - 91.5.

Fig. 8 shows histograms for the slopes of the troughs forming the peak between the peaks for HbA1c and A0, with measurements (82 measurements) of multiple HbE specimens using multiple apparatuses. Average: -4.2, standard deviation: 9.0, variance: 81.3, median: -5.0, maximum: 21.8, minimum: -25.9. Based on these conditions, the threshold range was set to -30 to 30, based on the maximum and minimum results. The reference threshold range may also be determined by statistical analysis, with a range of 95% assuming mean ±SD × constant and normal distribution.

Two different Hb-Riccarton-containing specimens were then measured, and since the slopes of the troughs forming the peak between the HbA1c and A0 peaks were -109 and -94, which were both below the previously established threshold values of -30, this demonstrated that a peak different from HbE had been accurately detected. The Hb-Riccarton-containing specimens (2 specimens) were also measured by affinity chromatography (HLC-723G8 affinity mode), which is thought to be less affected by abnormal hemoglobin, and values of 5.6% and 5.9% were obtained.

The same specimen was measured for HbAlc by the same calculation as HbE, with values of 5.3% and 5.6%, respectively.

When HbA1c was then measured while including the peak area between the peaks for HbA1c and A0 in the peak area for HbA1c, the values were 5.6% and 6.0%, respectively, which results were near the reference numerical values obtained by affinity chromatography.

The method of calculating HbE is a method of calculating the proportion (%) of HbAlc using the corrected value for the peak area of total hemoglobin (corrected area), obtained by subtracting the peak area for abnormal HbE from the peak area for total hemoglobin, and dividing the peak area for HbA1c by this corrected area.

### (Example 2)

This was carried out with an apparatus that did not have the reference threshold set in the initial state.

When measuring a specimen in which HbE was confirmed to be present, or a specimen with high accuracy, that information was registered in the device. The apparatus also stored the slope of the troughs forming the peak between the peaks for HbA1c and HbA0, as obtained by the measurement results. The apparatus also updated the reference threshold from previously accumulated slope information, and assessed unknown samples based on the reference threshold.

### REFERENCE SIGNS LIST

1 Analyzing means
2 Cation-exchange liquid chromatography apparatus
10 Eluent first solution
11 Eluent second solution
12 Eluent third solution
13 Degasser
14 Liquid feed pump
15, 16, 17 Solenoid valve
18 Autosampler
19 Analysis column
20 Detector
21 Column oven
22 Control/processing unit
23 Storage unit
24 Display screen (input device)
25 Data storage unit

## Claims

1. A method for analyzing hemoglobin from a blood-derived sample, the method comprising:
(1) a step of identifying a first peak for HbAlc and a second peak for HbA0 from a chromatogram representing elution of a plurality of hemoglobin fractions including at least HbAlc and HbA0, obtained by supplying a blood-derived sample to cation-exchange liquid chromatography;
(2) a step of discriminating whether or not an additional third peak is present between the first peak and the second peak, the following steps being further carried out when it has been judged that a third peak is present and no peaks other than the third peak are present;
(3) a step of identifying a first trough between the first peak and the third peak, and a second trough between the second peak and the third peak, and calculating the slope of a straight line formed between the first trough and the second trough; and
(4) a step of estimating the presence of abnormal hemoglobin in the sample based on comparison between the slope and a predetermined reference threshold range.

2. The method according to claim 1, wherein in step (3), the slope is calculated from the local minimum of the first trough and the local minimum of the second trough.

3. The method according to claim 1, wherein in step (4), it is estimated that abnormal hemoglobin different from hemoglobin E is present in the sample when the slope has deviated from the predetermined reference threshold range.

4. The method according to claim 3, wherein when it has been estimated that abnormal hemoglobin different from hemoglobin E is present in the sample, one or more steps are carried out from among the following steps:
(a) the response given is that the measurement results for HbAlc for the sample cannot be reported;
(b) the response given is the measurement results for HbAlc for the sample, with an associated identifier indicating the presence of the abnormal hemoglobin different from hemoglobin E; and
(c) the area of the third peak is included in the area of the first peak to calculate HbA1c, and the response given is the measurement results.

5. The method according to claim 4, wherein the abnormal hemoglobin different from hemoglobin E is Hb-Riccarton.

6. The method according to claim 1, wherein in step (4), it is estimated that hemoglobin E is present in the sample when the slope is within the predetermined reference threshold range.

7. The method according to claim 6,
wherein, when it is estimated that hemoglobin E is present in the sample, HbAlc is calculated by correcting hemoglobin E using the area of the third peak, and the response given is the measurement results.

8. The method according to claim 1, wherein the chromatogram is a chromatogram showing time on the abscissa and detector output on the ordinate, and the predetermined reference threshold range is -30 to 30, where the minimum detector output during measurement is normalized to 0 and the maximum value is normalized to 1000 in the chromatogram.

9. The method according to claim 1, wherein the predetermined reference threshold range is calculated by steps including the following steps:
(i) a step of identifying a first peak for HbA1c, a second peak for HbA0 and a third peak for hemoglobin E, from a chromatogram representing elution of a plurality of hemoglobin fractions including at least HbA1c, HbA0 and hemoglobin E, obtained by supplying a blood-derived sample of a hemoglobin E-containing reference specimen to cation-exchange liquid chromatography,
(ii) a step of identifying a first trough between the first peak and the third peak and a second trough between the second peak and the third peak, and calculating a reference slope from the local minimum of the first trough and the local minimum of the second trough; and
(iii) a step of carrying out (i) to (ii) for multiple hemoglobin E-containing reference specimens, and calculating the predetermined reference threshold range by statistical processing from the obtained values for the multiple reference slopes.

10. A hemoglobin analysis system in which the method according to claim 1 is carried out.

11. The hemoglobin analysis system according to claim 10, which comprises:
a cation-exchange liquid chromatography apparatus, and
analyzing means for analyzing chromatogram data obtained by the cation-exchange liquid chromatography apparatus.
